# EUROPEAN PATENT APPLICATION

(11) **EP 2 910 644 A1**
(43) Date of publication of application: **26.08.2015**
(21) Application number: 14000667.7
(22) Date of filing: 25.02.2014
(51) Int. Cl.: C12Q 1/37, C12Q 1/44

(54) **Method for the diagnosis of neuronal ceroid lipofuscinoses**

(71) Applicant: Steinbeis-Transferzentrum für Biopolymeranalytik und Biomolekül-Massenspektrometrie, 78464 Konstanz (DE); Universitätsklinikum Hamburg-Eppendorf (UKE), 20246 Hamburg (DE)
(72) Inventor: Przybylski, Michael, 65468 Trebur (DE); Cozma, Claudia, 78464 Konstanz (DE); Schulz, Angela, 22527 Hamburg (DE); Braulke, Thomas, 20251 Hamburg (DE)
(74) Representative: Müller-Boré & Partner Patentanwälte PartG mbB

(57) **Abstract**

The present invention relates to a method for the diagnosis of neuronal ceroid lipofuscinoses (NCLs), in particular CNL-1, CNL-2 and CNL-10, in a subject. The method is based on determining the activity of lysosomal enzymes with the help of umbelliferone derivatives and coumarin derivatives. The present invention further relates to said umbelliferone derivatives and coumarin derivatives, their use in the diagnosis of NCLs, and a kit for the diagnosis of NCLs.

## Description

The present invention relates to a method for the diagnosis of neuronal ceroid lipofuscinoses (NCLs) in a subject which is based on determining the activity of lysosomal enzymes with the help of umbelliferone derivatives and coumarin derivatives. The present invention further relates to said umbelliferone derivatives and coumarin derivatives, their use in the diagnosis of NCLs, and a kit for the diagnosis of NCLs.

Lysosomes, a kind of intracellular membrane vesicle, are responsible for the degradation and metabolism of many endogenous substances such as lipids, carbohydrates and proteins by the action of lysosomal enzymes. If, as a result of an inherited genetic defect or a biochemical defect, a lysosomal enzyme is absent or deficient regarding its function, lysosomal storage diseases (LSDs) are manifesting by several life-threatening symptoms.

LSDs are hereditary, mostly autosomal recessive metabolic disorders which can lead to severe and progressing disabilities and death. The symptoms of LSDs include mental retardation and bone, muscle and organ malformations, such as enlargement of the heart, liver and spleen, some of them being life-threatening already at early childhood. Approximately 60 to 70 different LSDs are known worldwide, including mucopolysaccharidoses, sphingomyelinoses, and NCLs. For several LSDs, treatment has recently become available by enzyme replacement therapy (ERT). However, for successful ERT it is critical to start the therapy early which necessitates an early diagnosis.

The neuronal ceroid lipofuscinoses (NCLs) are an etiologically and clinically heterogeneous group of autosomal recessive progressive pediatric encephalopathies, with a global incidence of 7 to 8 per 100,000 live births. These inherited diseases are characterized by dementia, epilepsy, and progressive physical decline, by profound neurodegeneration in the central nervous system and an early death of the patients. To date, at least 14 genetically different NCL forms have been identified. Intracellular localization and function (where known) of the defective NCL proteins are different: four NCL types are caused by defects in lysosomal enzymes (CLN-1, CLN-2, CLN-10, CLN-13), others by defects in transmembrane proteins (CLN-3, CLN-6, CLN-7, CLN-8). None of the NCL forms can be cured yet. However, a phase I/II clinical trial for intraventricular enzyme replacement therapy (ERT) has been developed for CLN2-disease for the first time. In this trial, CLN-2 patients undergo intraventricular application of recombinant CLN-2 enzyme via a Rickham capsule every 14 days. However, for the effective onset of ERT, early diagnosis is required, which render specific and sensitive diagnostics for neuronal ceroid lipofuscinoses of key importance. Rapid diagnostic methods of molecular specificity for NCLs have hitherto not been available.

As far as it is available, the gold standard in the diagnosis of NCLs today is the determination of the catalytic activity of affected lysosomal enzymes. This is mostly done by fluorimetric methods which allow a reliable diagnosis of particular NCLs. However, a fundamental drawback of these methods is the necessity to determine the activity of each relevant lysosomal enzyme one at a time. Since current fluorimetric methods mostly require the use of fresh blood samples, and said methods cannot be easily automated, the normal procedure is often to look for one specific NCL and, in case the result is negative, to look for another specific NCL and so on, until one particular NCL can be diagnosed. This procedure considerably prolongs the time until a reliable diagnosis can be established. Moreover, fluorimetric methods of diagnosis are only available for a limited range of known NCLs.

Accordingly, the technical problem underlying the present invention is to provide methods for the simple, fast, and accurate diagnosis of NCLs. Such methods should show a high sensitivity, need a minimal amount of patient sample material, and provide the ability to determine the activity of more than one lysosomal enzyme at the same time. Further, such methods should allow the actual determination to be effected by fluorimetry as well as mass spectrometry, since mass spectrometry, being extremely sensitive but also complex and expensive, could be used to validate fluorimetric methods which are more simple, economic and widespread.

The solution to the above technical problem is achieved by the embodiments characterized in the claims.

In particular, in a first aspect, the present invention relates to a method for the diagnosis of one or more neuronal ceroid lipofuscinoses, selected from the group consisting of CLN-1 (Infantile Neuronal Ceroid Lipofuscinose (INCL); Santavuori-Haltia Disease; Palmitoyl Protein Thioesterase Deficiency), CLN-2 (Late Infantile Neuronal Ceroid Lipofuscinose (LINCL); Jansky-Bielschowsky Disease; Tripeptidyl Peptidase deficiency), and CLN-10 (Cathepsin D Deficiency), in a subject, comprising the steps of:
(a) providing a blood sample of the subject;
(b) incubating the blood sample in the presence of
   (i) the palmitoyl protein thioesterase substrate 4-methyl-umbelliferyl-6-thiopalmitoyl-beta-glucoside, represented by formula (I) 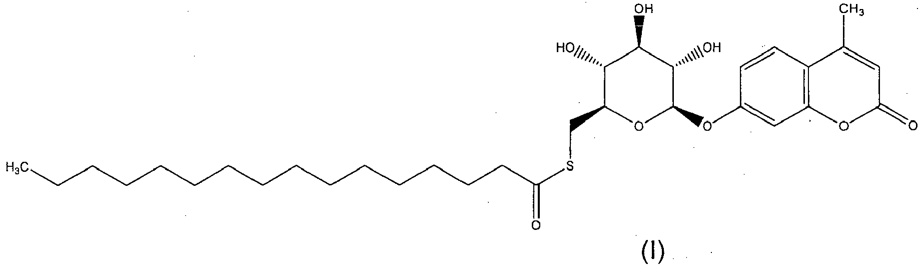 and/or
   (ii) a tripeptidyl peptidase substrate tripeptidyl-7-amido-4-methyl-coumarin, represented by formula (II) 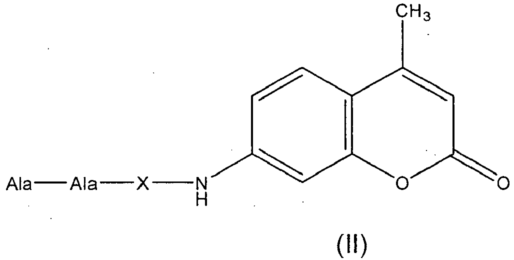 wherein
      Ala is L-alanine, and
      X is an amino acid, selected from the group consisting of L-alanine, L-valine, L-isoleucine, L-leucine, L-phenylalanine, L-tryptophane, L-methionine, L-proline, L-cysteine, L-arginine, and L-tyrosine; preferably from the group consisting of L-leucine, L-phenylalanine, and L-tyrosine;
         and/or
   (iii) a cathepsin D substrate dipeptidyl-7-amido-4-methyl-coumarin, represented by formula (III) 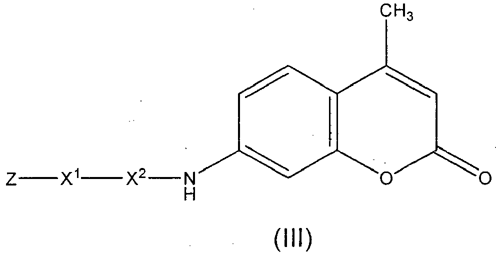 wherein
      X¹ and X² are amino acids, independently selected from the group consisting of L-alanine, L-valine, L-isoleucine, L-leucine, L-phenylalanine, L-tryptophane, L-methionine, L-proline, L-cysteine, L-arginine and L-tyrosine; preferably X¹ is selected from the group consisting of L-phenylalanine, L-arginine and L-tyrosine, and X² is L-phenylalanine; and
      Z is absent, or is selected from the group consisting of D-amino acids, or is N2-[(phenylmethoxy)carbonyl]-;
(c) in case the blood sample has been incubated in the presence of 4-methyl-umbelliferyl-6-thiopalmitoyl-beta-glucoside (step (b)(i)), incubating the blood sample in the presence of β-glucosidase;
(d) determining the amount of
   (i) 4-methyl-umbelliferone, represented by formula (IV) 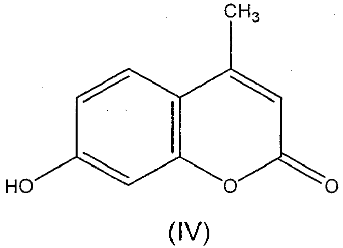 and/or
   (ii) 7-amino-4-methyl-coumarin, represented by formula (V) 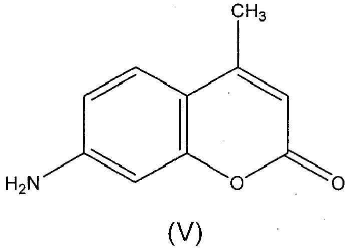 and/or
   (iii) X²-7-amino-4-methyl-coumarin, represented by formula (VI) 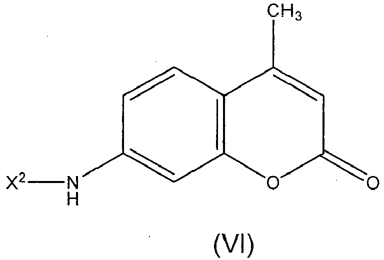
   wherein X² is the X² as defined above,
   in the sample;
   (e) assessing the activity of palmitoyl protein thioesterase and/or tripeptidyl peptidase and/or cathepsin D, based on the amounts of the compounds (i) and/or (ii) and/or (iii) as determined in step (d), respectively, wherein higher amounts of said compounds correlate with higher activities of the respective enzymes; and
   (f) assessing the presence or absence of CLN-1 and/or CLN-2 and/or CLN-10, wherein CLN-1 is present when the activity of palmitoyl protein thioesterase is absent or reduced in comparison to a healthy control subject, CLN-2 is present when the activity of tripeptidyl peptidase is absent or reduced in comparison to a healthy control subject, and CLN-10 is present when the activity of cathepsin D is absent or reduced in comparison to healthy control samples.

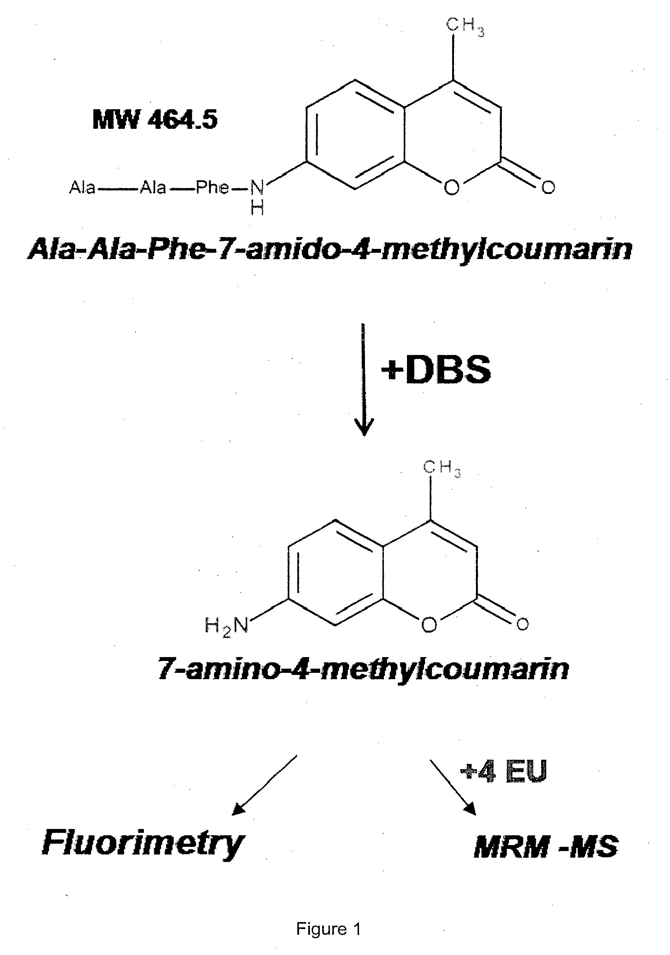

The method of the present invention is based on the specific cleavage of the above substrates (cf. step (b), (i) to (iii)) by the respective lysosomal enzymes. The products of this cleavage (cf. step (d), (i) to (iii)) are not endogenous blood compounds and are not further processed by lysosomal or other enzymes. Accordingly, they remain in solution after the enzymatic reaction and can be detected by fluorimetry or mass spectrometry (Fig. 1).

The neuronal ceroid lipofuscinoses that can be diagnosed with the method of the present invention are CLN-1, also known as Infantile Neuronal Ceroid Lipofuscinose (INCL), Santavuori-Haltia Disease, or Palmitoyl Protein Thioesterase Deficiency; CLN-2, also known as Late Infantile Neuronal Ceroid Lipofuscinose (LINCL), Jansky-Bielschowsky Disease, or Tripeptidyl Peptidase deficiency; and CLN-10, also known as Cathepsin D Deficiency. In this context, the acronyms CLN, NCL, and CNL are equally in use in the respective art, and are used herein in a synonymous manner. As an Example, the neuronal ceroid lipofuscinose CLN-1 as termed herein may be equally termed in the art as CNL-1, or NCL-1.

In preferred embodiments of the present invention, the subject for which a diagnosis should be established is a human subject.

The blood sample to be used in the method of the present invention can be any form of blood sample, e.g. whole blood, serum, plasma or dried blood spots (DBSs). Since DBSs only require a minimal amount of blood from the subject (about 100 µl fresh or EDTA-treated blood per spot), and can easily be stored at room temperature for a considerable amount of time, DBSs are particularly preferred.

Incubation as defined in step (b) of the method of the present invention is preferably incubation for 12 to 48 hours at a temperature of about 37°C. However, a person skilled in the art will know how this incubation can be modified, e.g. by incubating for a longer time at a lower temperature, or by incubating for a shorter time at a higher temperature, provided that said temperatures allow the proper functioning of the respective enzymes. Further, the exact duration of incubation can differ between different enzymes to be determined. Preferably, incubation is done in an aqueous medium.

The substrates to be used in the method of the present Invention are as defined above. Said substrates are sometimes referred to herein in connection with the specific NCL that can be assessed using said substrate. As an example, the substrate 4-methyl-umbelliferyl-6-thiopalmitoyl-beta-glucoside, which is specifically recognized by palmitoyl protein thioesterase, deficiency of which is the cause for CLN-1, can be referred to as "CLN-1 substrate" herein.

According to the method of the present invention, in case the blood sample has been incubated in the presence of 4-methyl-umbelliferyl-6-thiopalmitoyl-beta-glucoside (step (b)(i)), said method comprises further step (c) wherein the blood sample is further incubated in the presence of β-glucosidase, in order to cleave the β-glucoside moiety of the substrate. This incubation is preferably incubation for 1 to 8 hours at 37 °C. However, as indicated above, a person skilled in the art will know how this incubation can be modified. Further, this incubation is preferably done in an aqueous medium.

Step (d) of the method of the present invention, *i.e.* determining the amount of the products (cf. step (d), (i) to (iii)) generated by reaction of the respective substrates with the respective lysosomal enzymes, is done by fluorimetry, by mass spectrometry, or by both fluorimetry and mass spectrometry. In this context, mass spectrometry may preferably be multiple reaction monitoring-mass spectrometry (MRM-MS) or single reaction monitoring-mass spectrometry (SRM-MS). In particular, it is a notable feature of the method of the present invention that the above step can be effected by either fluorimetry or mass spectrometry, as well as by both methods in parallel. Since in both cases the same substrates are used, and the actual execution of the method of the present invention is largely identical up to the above step (d), results of either method can be easily compared and correlated to results of the other. Accordingly, the method of the present invention can e.g. be thoroughly validated by mass spectrometric determination, which is extremely sensitive, but also quite complex, costly and laborious, and the results obtained can be correlated to results obtained by fluorimetric determination, which is more simple, inexpensive and widespread, allowing e.g. the use of a thoroughly validated method also for laboratories that do not have access to mass spectrometry. In particular, the mass spectrometric method allows molecular determination with absolute specificity for each disease; further, it can be used for multiplex, *i.e*. duplex or triplex determinations.

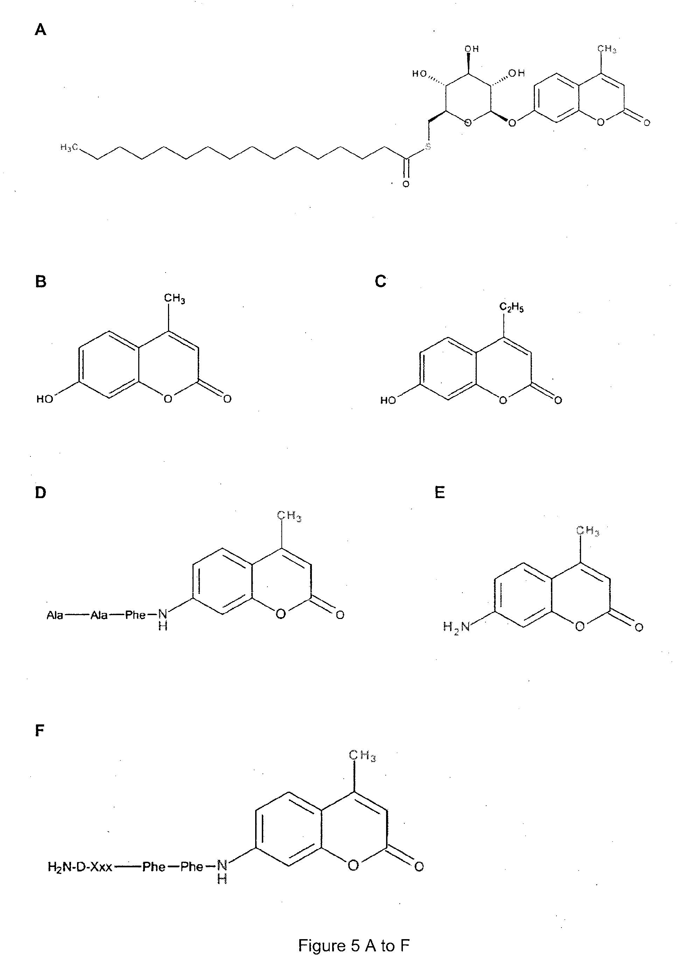

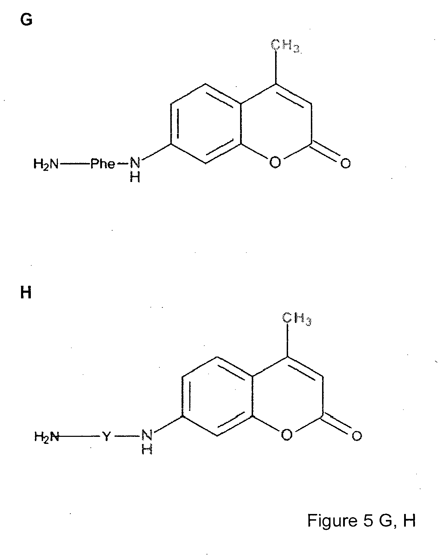

In the case of fluorimetric determination, a calibration curve is established using e.g. the standards shown in Table 1 and/or in Figure 5 in known concentrations. Fluorimetric determination can be done with any fluorimeter equipped with a specific umbelliferone filter. The excitation and emission wavelength of 4-alkyl umbelliferones, as well as 7-amino-4-alkyl coumarins are 360 and 400 nm, respectively. Suitable methods for the fluorimetric determination of 4-alkyl umbelliferones and 7-amino-4-alkyl coumarins are known in the art.

In the case of mass spectrometric determination, a suitable internal standard has to be used. Respective standards are known in the art. In a preferred embodiment, the standards shown in Table 1 and/or in Figure 5 are used. Parameters for mass spectrometric determinations are specific for different types of mass spectrometers, e.g. ion trap or triple-quadruple mass spectrometers, which can both be used in the method of the present invention, and for the type of measurement, e.g. single reaction monitoring mass spectrometry (SRM-MS) or multiple reaction monitoring mass spectrometry (MRM-MS) which both can be used in the method of the present invention. In a particular embodiment, MRM-MS is used. For all mass spectrometric assays, no additional sample cleanup is required. Accordingly, the incubation solution is mixed with the internal standard and directly injected into the electrospray source of the mass spectrometer. Suitable methods for the mass spectrometric determination of 4-alkyl umbelliferones, as well as 7-amino-4-alkyl coumarins, are known in the art.

In a particularly preferred embodiment of the method of the present invention, at least two of CLN-1, CLN-2, and CLN-10 are assessed, or all three of CLN-1, CLN-2, and CLN-10 are assessed. For each disease to be assessed, the respective substrate is incubated with the sample in step (b) of the method of the present invention, the respective reaction product is determined in step (d) of the method of the present invention, and the activity of the respective enzyme is assessed in step (e) of the method of the present invention. In this "multiplex", *i.e.* duplex or triplex mode of the method of the present invention, the activity of more than one respective lysosomal enzyme can be determined in one sample, satisfying the long-felt need to screen for more than one NCL at the same time and in the same sample.

In the method of the present invention, the activity of lysosomal enzymes, deficiency of which is the cause for various NCLs, can be determined. In case the activity of a particular enzyme is determined to be 20% lower than the average activity of said enzyme in healthy control samples, the corresponding NCL is assessed to be present.

The method of the present invention will enable a substantial extension and improvement of previously employed NCL diagnostics, and will lead to a rapid, reliable and widely applicable spectrum of clinical diagnostics for said diseases. A major advantage of the present invention is the common substrates both for fluorimetric and mass spectrometric determination of enzyme activity. This feature enables the easy correlation of data obtained e.g. by different laboratories, regardless of the fact which of the two determination methods have been used. Further, the method of the present invention is able to distinguish between healthy subjects, (heterozygous) carriers of the disease, and (homozygous) subjects suffering from the disease.

In a further aspect, the present invention relates to 4-methyl-umbelliferyl-6-thiopalmitoyl-beta-glucoside, represented by formula (I). 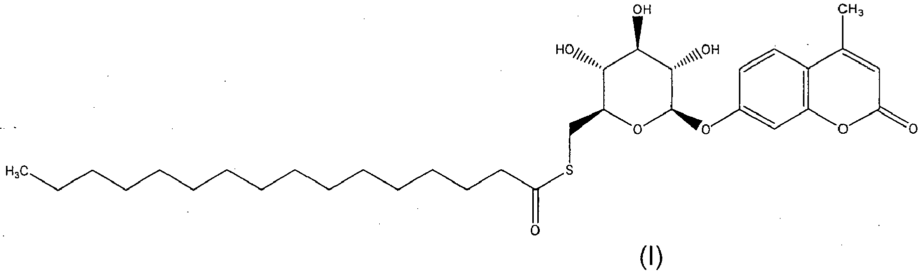 In a further aspect, the present invention relates to a tripeptidyl-7-amido-4-methyl-coumarin, represented by formula (II) 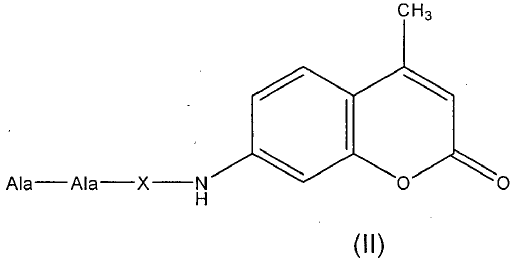 wherein Ala is L-alanine, and X is an amino acid, selected from the group consisting of L-alanine, L-valine, L-isoleucine, L-leucine, L-phenylalanine, L-tryptophane, L-methionine, L-proline, L-cysteine, L-arginine, and L-tyrosine; preferably from the group consisting of L-leucine, L-phenylalanine, and L-tyrosine.

In a further aspect, the present invention relates to a dipeptidyl-7-amido-4-methyl-coumarin, represented by formula (III) 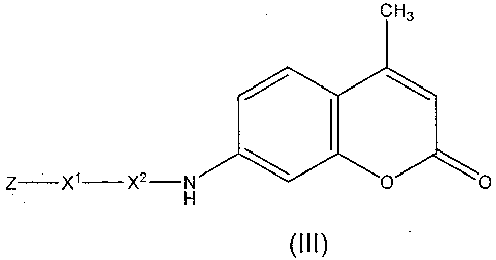 wherein
X¹ and X² are amino acids, independently selected from the group consisting of L-alanine, L-valine, L-isoleucine, L-leucine, L-phenylalanine, L-tryptophane, L-methionine, L-proline, L-cysteine, L-arginine and L-tyrosine; preferably X¹ is selected from the group consisting of L-phenylalanine, L-arginine and L-tyrosine, and X² is L-phenylalanine; and
Z is absent, or is selected from the group consisting of D-amino acids, or is N2-[(phenylmethoxy)carbonyl]-;

In preferred embodiments, the above compounds are for use in the diagnosis of one or more neuronal ceroid lipofuscinoses, selected from the group consisting of CLN-1 (Infantile Neuronal Ceroid Lipofuscinose (INCL); Santavuori-Haltia Disease; Palmitoyl Protein Thioesterase Deficiency), CLN-2 (Late Infantile Neuronal Ceroid Lipofuscinose (LINCL); Jansky-Bielschowsky Disease; Tripeptidyl Peptidase deficiency), and CLN-10 (Cathepsin D Deficiency).

In a final aspect, the present invention relates to a kit for the diagnosis of one or more neuronal ceroid lipofuscinoses, selected from the group consisting of CLN-1 (Infantile Neuronal Ceroid Lipofuscinose (INCL); Santavuori-Haltia Disease; Palmitoyl Protein Thioesterase Deficiency), CLN-2 (Late Infantile Neuronal Ceroid Lipofuscinose (LINCL); Jansky-Bielschowsky Disease; Tripeptidyl Peptidase deficiency), and CLN-10 (Cathepsin D Deficiency), in a subject, comprising one or more of the compounds as defined above.

In a preferred embodiment, the kit of the present invention further comprises standards for the fluorimetric and/or mass spectrometric determination as defined above for the method of the present invention. Moreover, the kit of the present invention can further comprise suitable buffers, solutions, reagents and disposables.

In the present invention, a new, general type of substrate derivatives for the diagnosis of NCLs has been developed, based on alkyl-umbelliferone analogs used as building blocks with different substitutions. These substrate derivatives enable specific fluorimetric diagnostics, as well as mass spectrometric diagnostics (*multiple reaction monitoring-mass spectrometry*; MRM-MS; or *single reaction monitoring-mass spectrometry*; SRM-MS), using the same identical substrate derivative. In the case of mass spectrometric determination, the use of multiple 4- or 7-alkyl-umbelliferone derivatives with different alkyl substituents as standards provides the possibility of multiplex mass spectrometric diagnostics. Here, the specific and highly sensitive clinical diagnosis on dry blood spots (DBS) for NCLs by fluorimetric and/or MRM-MS determination or in multiplex assays by MRM-MS analyses using newly developed, specific substrate derivatives and coumarin derivatives suitable as internal standards is described. Chemical formulae of exemplary substrates and standard samples are shown in Table 1.

**Table 1: Exemplary substrates and internal standards**

| **CLN** | **Substrate** | **Product** | **External standards (fluorimetry)** | **internal standards (MRM-MS)** |
|---|---|---|---|---|
| CLN-1 | *4-methyl-umbellif eryl-6-thiopalmito yl-beta-glucoside* | *4-methyl-umbellife rone* | *4-methyl-umbell iferone* | *4-ethyl-umbellife rone* |
| Palmitoyl protein thioesterase deficiency | | | | |
| CLN-2 | *Ala-Ala-Phe-7-am ido-4-methylcoum arin* | *7-amino-4-methyl-coumarin* | *7-amino-4-meth yl-coumarin* | *4-ethyl-umbellife rone* |
| Tripeptidyl peptidase deficiency | | | | |
| CLN-10 | *Z-Phe-Phe-7-ami do-4-methyl-coum arin* | *H-Phe-7-amino-4-methyl-coumarin* | *H-Phe-7-amino-4-methyl-coum arin* | *H-Tyr-7-amino-4 -methyl-coumari n* |
| Cathepsin D deficiency | | | | |

| | | | | |
|---|---|---|---|---|
| **** Chemical formulae are shown in*** ***Figure 5*** | | | | |

The figures show:
Figure 1:
   Substrate conversion of the CLN-2 substrate of the present invention in dried blood spot (DBS).
Figure 2:
   Tripeptidyl peptidase activity determination by MS-MRM for CLN-2 patients and healthy donors.
Figure 3:
   Example of tripeptidyl peptidase activity determination by MRM-MS for CLN-2 patient (a.) vs. healthy donor (b.). P, reaction product or 7-amino-4-methyl-coumarin; IS - internal standard 4-ethyl-umbelliferone.
Figure 4:
   Schematic representation of the MRM-MS CLN-1, CLN-2 and CLN-10 triplex assay according to the present invention.
Figure 5:
   Chemical formulae of CLN substrates, products and standards. CLN-1 Substrate: 4-methyl-umbelliferyl-6-thiopalmitoyl-beta-glucoside (A). CLN-1 product and internal standard: 4-methyl-umbelliferone (B). CLN-1 and CLN-2 internal standard for MRM-MS determination: 4-ethyl-umbelliferone (C). CLN-2 substrate: Ala-Ala-Phe-7-amido-4-methylcoumarin (D). CLN-2 product and external standard for fluorimetry: 7-amino-4-methyl-coumarin (E). CLN-10 substrate: D-Xxx-Phe-Phe-7-amido-4-methyl-coumarin (where D-Xxx is a D-amino acid (F). CLN-10 product and internal standard for fluorimetry: H-Phe-7-amino-4-methyl-coumarin (G). CLN-10 internal standard for MRM-MS determination: H-Tyr-7-amino-4-methyl-coumarin (H).

The present invention will now be further illustrated in the following examples without being limited thereto.

### Examples

### Example 1:

### Description of assay for diagnosis of CLN-1, CLN-2 and CLN-10 in single determinations by fluorimetry and mass spectrometry.

Standard protocol for CLN single diagnostic assay:
1. DBS (Dry-blood spot filter cards): Samples are obtained with a standard puncher (3 mm diameter); Filter card: Whatman filter 903.
2. Extraction of blood samples: on the DBS 60 µL buffer are introduced (20 mM ammonium acetate buffer, pH 4.5, containing 0.02% NaN₃), agitate for 45 minutes at 25 °C, take 50 µL extract and introduce them into a new well of a 96 well plate (opaque, standard for fluorimetry determinations).
3. Incubation with substrate: on top of the fresh blood extract, 50 µL substrate solution (500 µM) in water, was prepared as follows: A standard solution of 100 mM in DMSO is prepared and directly before the incubation for the assay dilution to 1:200 carried out, and added on top of the blood extract. The plate is then sealed with a plate sealer, covered in aluminum foil for subdued light, and incubated for 17 h at 37 °C under agitation.

Substrates, as well as formed products, are listed in Table 1. Further, Figure 5 shows a compilation of substrate derivatives.
4. Quenching of the reaction: (i) For CLN-2 and CLN-10 determination, the reaction is quenched by addition of 10 µL formic acid; (ii) For determination of CLN-1 substrate, conversion is continued with a second step by addition of beta-glucosidase (equivalent of 2 U in 20 µL reaction buffer (10 mM ammonium acetate, pH 4.5, containing 0.02 % NaN₃) for additional 3 h under agitation at 37 °C. After this second incubation, the reaction is quenched with 10 µL formic acid.
5. Fluorimetric determination: Fluorimetric assays are performed with a Victor-2 plate reader (Beckman) using a standard umbelliferone filter at 2 time points: (i) Before incubation (used as reference; the values are extracted from the final measurements); (ii) after quenching the reaction.
6. Mass spectrometric (SRM-MS or MRM-MS) determination. Sample preparation for MS is carried out with 50 µL of internal standard solution added at a concentration of 15 µmol; the sample is then centrifuged at 130000 rpm for 5 minutes, and 50 µL supernatant used for the mass spectrometric determination. All coumarin derivatives have the same MS-MS fragmentation pattern. Measurements are carried out by (i) SRM-MS (*single reaction monitoring-mass spectrometry*) measurement of parent ions, or (ii) by MRM-MS of the transition parent ion (M) to daughter ion
   (M-28 or M-44)⁺.
7. Data evaluation. Fluorimetric and mass spectrometric determinations provide relative concentrations of products, which are transformed into enzymatic activity, nmol / mL blood / h. All determinations are performed in triplicate, with standard protocol for statistical evaluation.

### Example 2:

### Example of CLN determination in single assay - Tripeptidyl peptidase activity (CLN-2) determination in patient samples and healthy donors.

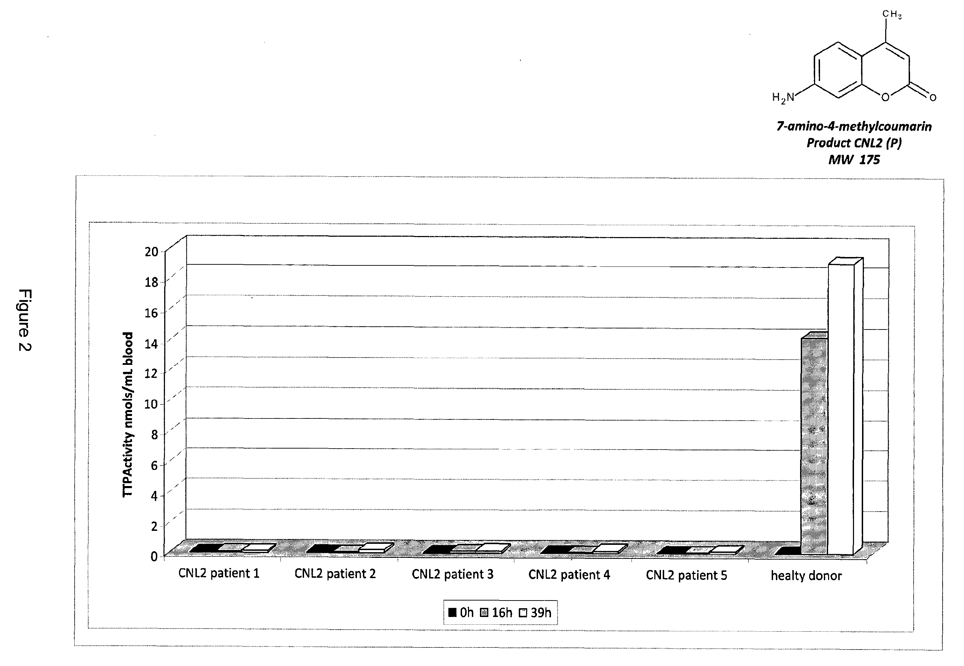

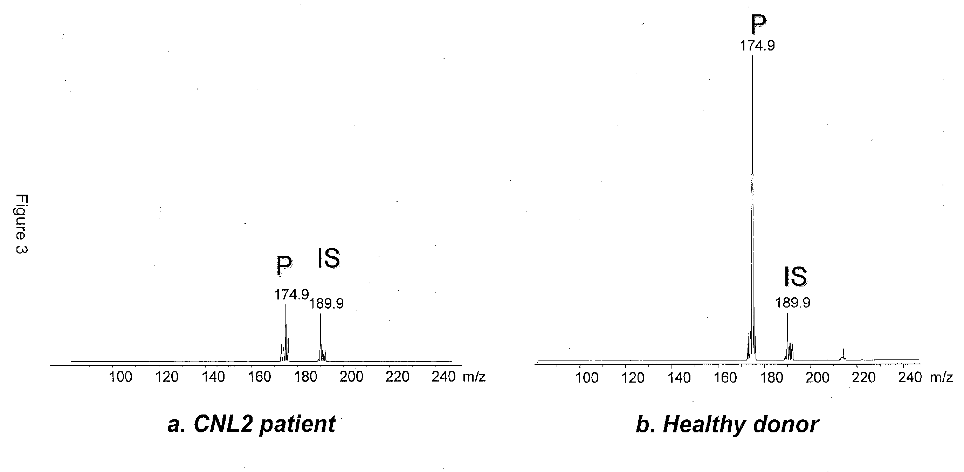

Tripeptidyl peptidase activity was determined in DBS samples for 6 CLN-2 patients (positive controls), and 8 healthy controls, according to the assay described above, by determination of the conversion rate of CLN-2 substrate into 7-amino-4-methyl-coumarin, as in the reaction shown in Figure 1. The assays showed for both the fluorimetric and mass spectrometric determination corresponding values, with a unequivocal difference between CLN-2 patient samples and healthy donors in enzymatic activity of tripeptidyl peptidase. Representative examples are shown in Figures 2 and 3.

### Example 3:

### Example of mass spectrometric triplex determination of CLN-1, CLN-2 and CLN-10.

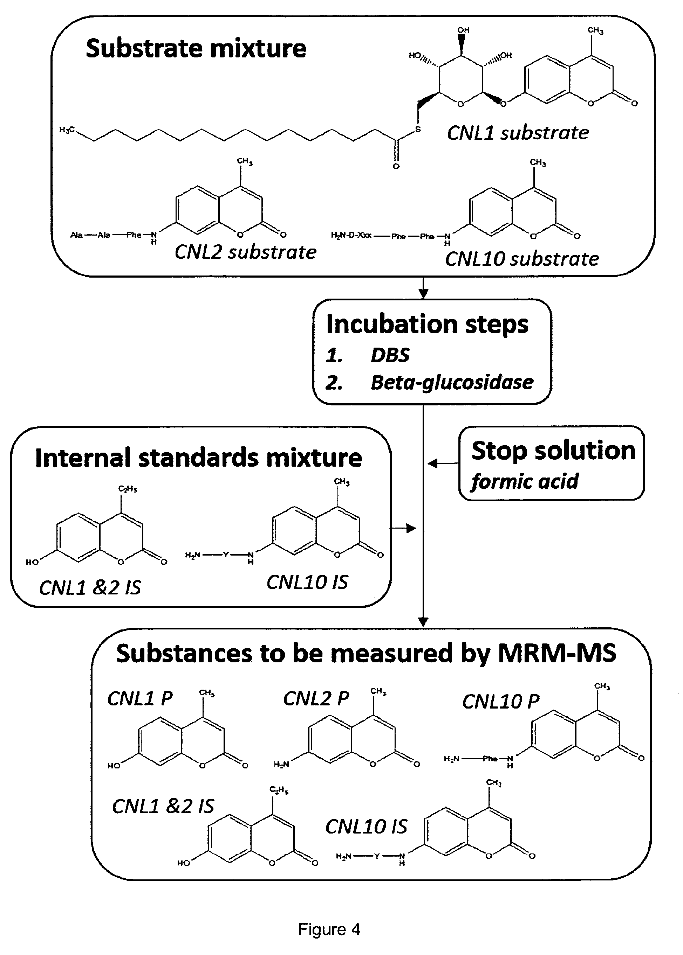

Standard protocol for CLN-1, CLN-2 and CLN-10 triplex assay:
1. DBS samples are obtained with a standard puncher (3 mm diameter) on Whatman filter 903 paper.
2. Extraction of blood: To the DBS, 60 µL buffer (20 mM ammonium acetate buffer, pH 4.5, containing 0.02 % NaN₃) are added under agitation for 45 minutes at 25 °C. A 50 µL extract aliquot is then introduced into a new well of a 96 well plate system (standard as used for fluorimetric determinations).
3. Incubation with the substrates: On top of the fresh blood extract, 50 µL substrate solution (500 µM CLN-1 substrate, 500 µM CLN-2 substrate and 500 µM CLN-10 substrate prepared in water) are introduced. The substrates, as well the expected products are listed in Table 1. The plate is sealed with a plate sealer, covered in aluminum foil and incubated under agitation for 17 h at 37 °C.
4. Incubation with supplementary enzyme for CLN-1. Beta-glucosidase is added (equivalent of 2 U) in 35 µL reaction buffer (10 mM ammonium acetate buffer, pH 4.5, containing 0.02 % NaN₃). The plate is then resealed and incubated for 3 h under agitation at 37 °C.
5. Quenching of the reaction: The reaction is quenched with addition of 15 µL formic acid.
6. Mass spectrometry (MS) determination. Sample preparation for MS is carried out by addition of 50 µL internal standard solution with a concentration of 15 µM, followed by centrifugation of the sample at 130000 rpm for 5 minutes. An aliquot of 50 µL supernatant is used for mass spectrometric determination. All coumarin derivatives have the same fragmentation pattern, enabling measurement by SRM-MS of the parent ions, or by MRM-MS of the transition of parent ion (M) to daughter ion (M-28 or M-44). A schematic presentation of the triplex assay is shown in Figure 4.
7. Data evaluation. Enzymatic activities are expressed in nmol / mL blood / h.

## Claims

1. A method for the diagnosis of one or more neuronal ceroid lipofuscinoses, selected from the group consisting of CLN-1 (Infantile Neuronal Ceroid Lipofuscinose (INCL); Santavuori-Haltia Disease; Palmitoyl Protein Thioesterase Deficiency), CLN-2 (Late Infantile Neuronal Ceroid Lipofuscinose (LINCL); Jansky-Bielschowsky Disease; Tripeptidyl Peptidase deficiency), and CLN-10 (Cathepsin D Deficiency), in a subject, comprising the steps of:
(a) providing a blood sample of the subject;
(b) incubating the blood sample in the presence of
(i) the palmitoyl protein thioesterase substrate 4-methyl-umbelliferyl-6-thiopalmitoyl-beta-glucoside, represented by formula (I) 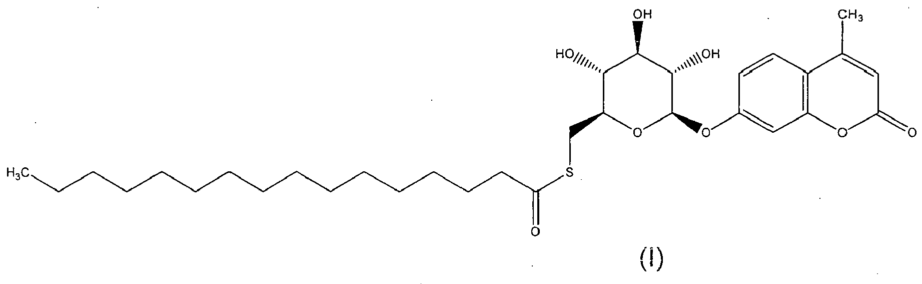 and/or
(ii) a tripeptidyl peptidase substrate tripeptidyl-7-amido-4-methyl-coumarin, represented by formula (II) 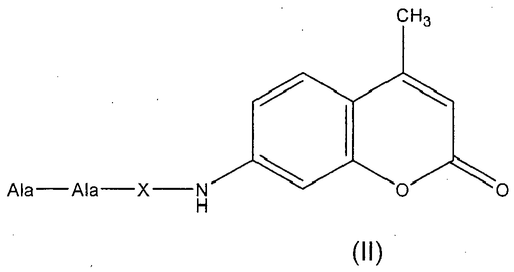 wherein
Ala is L-alanine, and
X is an amino acid, selected from the group consisting of L-alanine, L-valine, L-isoleucine, L-leucine, L-phenylalanine, L-tryptophane, L-methionine, L-proline, L-cysteine, L-arginine, and L-tyrosine,
and/or
(iii) a cathepsin D substrate dipeptidyl-7-amido-4-methyl-coumarin, represented by formula (III) 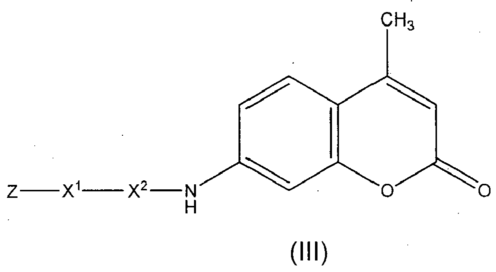 wherein
X¹ and X² are amino acids, independently selected from the group consisting of L-alanine, L-valine, L-isoleucine, L-leucine, L-phenylalanine, L-tryptophane, L-methionine, L-proline, L-cysteine, L-arginine and L-tyrosine; and
Z is absent, or is selected from the group consisting of D-amino acids, or is N2-[(phenylmethoxy)carbonyl]-;
(c) in case the blood sample has been incubated in the presence of 4-methyl-umbelliferyl-6-thiopalmitoyl-beta-glucoside (step (b)(i)), incubating the blood sample in the presence of β-glucosidase;
(d) determining the amount of
(i) 4-methyl-umbelliferone, represented by formula (IV) 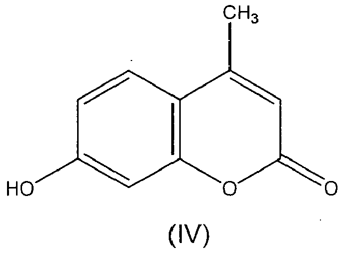 and/or
(ii) 7-amino-4-methyl-coumarin, represented by formula (V) 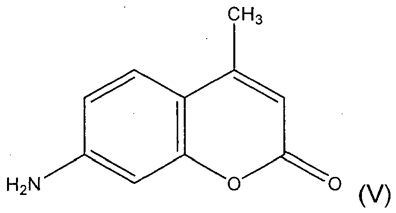 and/or
(iii) X²-7-amino-4-methyl-coumarin, represented by formula (VI) 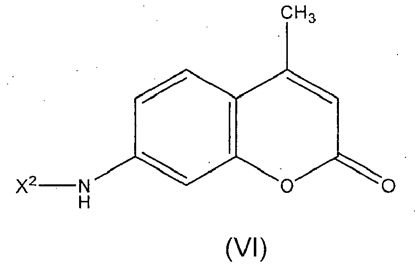 wherein X² is the X² as defined above,
in the sample;
(e) assessing the activity of palmitoyl protein thioesterase and/or tripeptidyl peptidase and/or cathepsin D, based on the amounts of the compounds (i) and/or (ii) and/or (iii) as determined in step (d), respectively, wherein higher amounts of said compounds correlate with higher activities of the respective enzymes; and
(f) assessing the presence or absence of CLN-1 and/or CLN-2 and/or CLN-10, wherein CLN-1 is present when the activity of palmitoyl protein thioesterase is absent or reduced in comparison to a healthy control subject, CLN-2 is present when the activity of tripeptidyl peptidase is absent or reduced in comparison to a healthy control subject, and CLN-10 is present when the activity of cathepsin D is absent or reduced in comparison to healthy control samples.

2. The method of claim 1, wherein the blood sample is selected from the group consisting of whole blood, serum, plasma, and dried blood spots (DBS).

3. The method of claim 1 or claim 2, wherein the incubation in step (b) is for 12 to 48 hours at 37 °C.

4. The method of any one of claims 1 to 3, wherein the incubation in step (c) is for 1 to 8 hours at 37 °C.

5. The method of any one of claims 1 to 4, wherein determining the amount of compounds (i) and/or (ii) and/or (iii) in step (d) is done by fluorimetry, mass spectrometry, or both fluorimetry and mass spectrometry.

6. The method of any one of claims 1 to 5, wherein at least two of CLN-1, CLN-2, and CLN-10 are assessed, or wherein all three of CLN-1, CLN-2, and CLN-10 are assessed.

7. The method of any one of claims 1 to 6, wherein CLN-1 and/or CLN-2 and/or CLN-10 is assessed to be present when the activity of the respective enzyme is at least 20% lower than the average activity of said enzyme in healthy control samples.

8. 4-methyl-umbelliferyl-6-thiopalmitoyl-beta-glucoside, represented by formula (I).

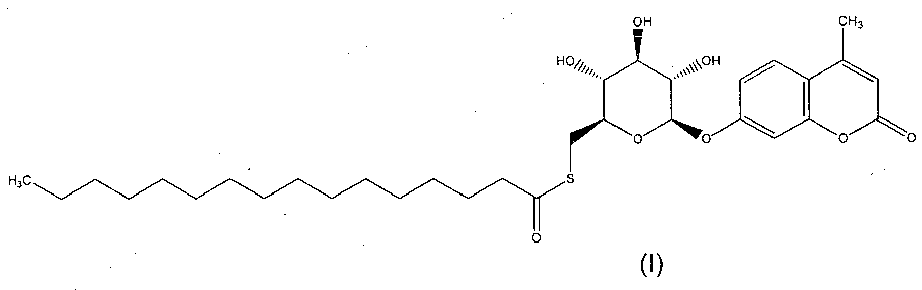

9. A tripeptidyl-7-amido-4-methyl-coumarin, represented by formula (II) 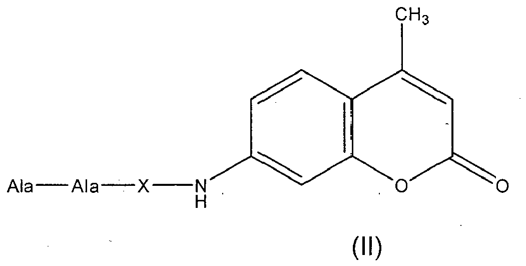 wherein
Ala is L-alanine, and
X is an amino acid, selected from the group consisting of L-alanine, L-valine, L-isoleucine, L-leucine, L-phenylalanine, L-tryptophane, L-methionine, L-proline, L-cysteine, L-arginine, and L-tyrosine.

10. A dipeptidyl-7-amido-4-methyl-coumarin, represented by formula (III) 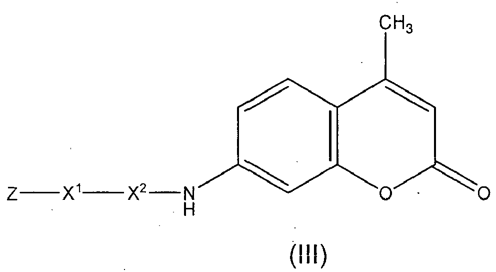 wherein
X¹ and X² are amino acids, independently selected from the group consisting of L-alanine, L-valine, L-isoleucine, L-leucine, L-phenylalanine, L-tryptophane, L-methionine, L-proline, L-cysteine, L-arginine and L-tyrosine; and
Z is absent, or is selected from the group consisting of D-amino acids, or is N2-[(phenylmethoxy)carbonyl]-.

11. The compounds of any one of claims 8 to 10 for use in the diagnosis of one or more neuronal ceroid lipofuscinoses, selected from the group consisting of CLN-1 (Infantile Neuronal Ceroid Lipofuscinose (INCL); Santavuori-Haltia Disease; Palmitoyl Protein Thioesterase Deficiency), CLN-2 (Late Infantile Neuronal Ceroid Lipofuscinose (LINCL);
Jansky-Bielschowsky Disease; Tripeptidyl Peptidase deficiency), and CLN-10 (Cathepsin D Deficiency).

12. A kit for the diagnosis of one or more neuronal ceroid lipofuscinoses, selected from the group consisting of CLN-1 (Infantile Neuronal Ceroid Lipofuscinose (INCL); Santavuori-Haltia Disease; Palmitoyl Protein Thioesterase Deficiency), CLN-2 (Late Infantile Neuronal Ceroid Lipofuscinose (LINCL); Jansky-Bielschowsky Disease; Tripeptidyl Peptidase deficiency), and CLN-10 (Cathepsin D Deficiency), in a subject, comprising one or more of the compounds as defined in claims 8 to 10.
